# EUROPÄISCHE PATENTSCHRIFT

(11) **EP 0 347 873 B1**
(45) Veröffentlichungstag und Bekanntmachung des Hinweises auf die Patenterteilung: **30.08.1995**
(21) Anmeldenummer: 89111275.7
(22) Anmeldetag: 21.06.1989
(51) Int. Cl.: C12P 1/00, C12P 7/24, C12P 13/00

(54) **Verfahren zur biokatalytischen herstellung sclecht Wasserlöslicher Substanzen mit integrierter extraktiver Aufarbeitung**
Process for the biocatalytic production of poorly water soluble compounds with integrated extraction
Procédé pour la production biocatalytique de compositions peu solubles dans l'eau avec traitement d'extraction intégré

(30) Priorität: 23.06.1988 DD 317055
(43) Veröffentlichungstag der Anmeldung: 27.12.1989
(73) Patentinhaber: Degussa Aktiengesellschaft, 60311 Frankfurt (DE)
(72) Erfinder: Miethe, Peter, Dr.rer.nat., DDR-4020 Halle (Saale) (DD); Voss, Harald, Prof.Dr.sc.nat., DDR-4090 Halle-Neustadt (DD); Gruber, Ronald, Dipl.-Chem., DDR-4020 Halle (Saale) (DD); Poetzsch, Helga, Dipl.-Ing., DDR-4070 Halle (Saale) (DD)

(56) Entgegenhaltungen:
- EP-A- 0 118 928
- EP-A- 0 340 744
- EUR. J. BIOCHEM., Band 161, 1986, Seiten 149-154; N.L. KLYACHKO et al.: "Catalysis by enzymes entrapped into hydrated surfactant aggregates having lamellar or cylindrical (hexagonal) or ball-shaped (cubic) structure in organic solvents"

## Beschreibung

Die Erfindung betrifft ein biokatalytisches Verfahren mit integrierter extraktiver Produktaufarbeitung. Das Verfahren ist geeignet für den Umsatz organischer Substrate und die Herstellung von Produkten, die in organischen Lösungsmitteln löslich sind.

Biokatalytische Verfahren, die mit einer Produktextraktion gekoppelt sind, besitzen den prinzipiellen Vorteil, daß eine Produktinhibierung umgangen werden kann. Darüber hinaus kann eine hohe Raum-Zeit-Ausbeute durch kontinuierliche und halbkontinuierliche Prozeßführung erreicht werden. Für wäßrige Zweiphasensysteme sind derartige Verfahren gut bekannt.

Diese Verfahren sind jedoch nur dann praktikabel, wenn die eingesetzten Substrate und Produkte ausreichend wasserlösich sind. Zur Extraktion hydrophober Produkte sind zweiphasige flüssige Systeme, bestehend aus organischem Lösungsmittel und einer den Biokatalysator enthaltenden wäßrigen Phase vorgeschlagen worden. Diese Verfahrensweise ist problematisch, da es an der Grenzschicht zwischen organischem Lösungsmittel und Wasser zur Enzymdenaturierung und zur Schädigung der eingesetzten Mikroorganismen und Zellen kommt. Weisen darüber hinaus auch die eingesetzten Substrate nur eine geringe Wasserlöslichkeit auf, lassen sich mit dem Verfahren nur sehr geringe Umsatzraten erzielen. Für den Umsatz hydrophober Substrate besitzen reversmizellare Phasen sehr günstige Eigenschaften.

Ein flüssiges Zweiphasensystem, das eine Extraktion ermöglicht, ist in der Form bekannt, daß eine reversmizellare Phase mit reinem Wasser koexistiert. Damit ist prinzipiell die Möglichkeit eines Umsatzes hydrophober Substrate und der Extraktion hydrophiler Produkte gegeben.

Es ist auch bekannt (Eur. J. Biochem., 6 (1986), 149 - 54), Biokatalysatoren in Dreikomponentensystemen aus Wasser/organischem Lösungsmittel/Tensid in flüssigkristallinen Phasen zu solubilisieren. Die untersuchten Enzyme zeigten Aktivität in flüssigkristallinen Mesophasen, wobei die Aktivität von der speziellen flüssigkristallinen Struktur abhing.

Das Ziel der Erfindung besteht in der Kopplung einer Biokatalyse, bei der hydrophile oder hydrophobe Substrate umgesetzt werden, mit einer Extraktion, bei der hydrophobe Produkte durch ein organisches Lösungsmittel kontinuierlich extrahiert werden können, wobei durch die Extraktion keine übermäßige Desaktivierung des Biokatalysators verursacht wird.

Diese Aufgabe besteht in der Nutzung der biokatalytischen Eigenschaften von isolierten Enzymen, sowie von Enzymsystemen pro- und eukariotischer Zellen in einem flüssigem System, welches für die kontinuierliche Produktextraktion eine organische Lösungsmittelphase enthält.

Erfindungsgemäß werden in Tensidphasen solubilisierte Biokatalysatoren in einem Zweiphasengebiet eines Dreikomponentengemisches, bestehend aus Wasser, organischem Lösungsmittel und Tensid, eingesetzt. In diesem Zweiphasengebiet koexistiert die biokatalysatorhaltige Tensidphase mit reinem organischen Lösungsmittel. Die Substratzuführung kann entweder über die organische Lösungsmittelphase erfolgen oder das Substrat wird in fester Form zugeführt und direkt in der Tensidphase gelöst. Dabei wird das gute und sehr komplexe Lösungsvermögen der Tensidphase sowohl für hydrophile als auch für hydrophobe Substanzen genutzt. Die entstehenden hydrophoben Produkte werden von der koexistierenden organischen Lösungsmittelphase extrahiert und können in einem Folgeschritt aufgearbeitet werden. Als organische Lösungsmittel können z.B. Hexan, Heptan, Octan, Cyclohexan, Benzol oder Mischungen dieser Lösungsmittel mit Aceton, Acetessigester oder ähnliche Lösungsmittelgemische eingesetzt werden.

Als Tenside können anionische Amphiphile, wie Alkalialkylsulfate, Alkalialkylsulfonate, Alkalialkylcarboxylate, Alkalialkylarylsulfonate, Alkalidialkylsulfosuccinate, Erdalkalidialkylsulfosuccinate; oder ein kationisches Amphiphil, wie Alkylammoniumsalze, Alkylpyridiniumsalze, Alkyltrimethylammoniumsalze; oder ein zwitterionisches Amphiphil, wie ein Phospholipid, Sulfobetain, Carbobetain; oder ein nichtionisches Amphiphil, wie Polyoxyethylenether, Polyoxyethylenester, Polyoxyethylensorbitanester, Alkylphenolpolyethylenglykolester oder entsprechende Tensidgemische verwendet werden.

Als Biokatalysatoren können neben isolierten Enzymen, wie Alkoholdehydrogenase, Chymotrypsin, Lipase, auch pro- und eukariotische Zellen, beispielsweise von E. coli oder Saccaromyces cerevisiae, auch Enzyme und Zellen gleichzeitig eingesetzt werden.

Die Tensidphase, die in der kolloidchemischen Literatur auch als Mittelphase bezeichnet wird, ist flüssig und niedrigviskos. In einigen Fällen bilden Tensidphase und reversmizellare bzw. mizellare Phase mikroskopisch ein einheitliches Zustandsgebiet aus. Auch in dieser Phase kann der Biokatalysator solubilisiert werden. Biokatalysatoren, d.h. isolierte Enzyme oder ganze Zellen, zeigen in diesen Phasen eine bemerkenswert hohe biokatalytische Aktivität, die auch nach längerer Lagerzeit erhalten bleibt. Eine Zellvermehrung findet unter diesen Umständen in der Regel nicht oder nur in sehr eingeschränktem Maße statt.

Bei Verwendung des zweiphasigen Reaktionssystems, bestehend aus biokatalysatorhaltiger Tensidphase und organischer Lösungsmittelphase, werden die praktischen Möglichkeiten für die biokatalytische Synthese von organischen Substanzen, wie Steroiden, Lipiden, langkettigen aliphatischen und aromatischen Alkoholen, Aldehyden, Carbonsäuren und Estern, wesentlich erweitert. Darüberhinaus ergibt sich auch die Möglichkeit einer Phasentransferkatalyse bei der Peptidsynthese.

Die Biokatalyse ist in jedem Falle vom Verteilungsgleichgewicht der Substrate und Produkte zwischen den koexistierenden Phasen bzw. vom Lösungsverhalten der Substrate in der Tensidphase beeinflußt.

Technische Synthesen auf Basis des vorgeschlagenen Verfahrens können in einfachen Reaktions- und Extraktionsapparaturen, z.B. Rührkesseln, Extraktionskolonnen oder Mixer-Settler-Einheiten, ausgeführt werden. Dabei ist auch eine kontinuierliche Prozeßführung möglich.

### Ausführungsbeispiele:

### Beispiel 1:

Ein zweiphasiges flüssiges System kann auf folgende Weise hergestellt werden:
Eine 8 gewichtsprozentige Lösung von Polyoxyethylen (9,7) nonylphenylether in Cyclohexan wird hergestellt. 114 ml dieser Lösung werden mit 20 ml Wasser versetzt. Nach dem Schütteln und Zentrifugieren bilden sich zwei klare flüssige Phasen - die obere besteht aus nahezu reinem Hexan, die untere ist die Tensidphase.

### Beispiel 2:

Es wird eine 10 gewichtsprozentige Lösung von Polyoxyethylen (9) heptylphenylester in Hexan hergestellt. Zu 1 l dieser Lösung werden 10 ml einer 21 mM wäßrigen NAD⁺ -Lösung gegeben. Dann werden 5 ml einer wäßrigen Lösung von Hefe-Alkoholdehydrogenase eingespritzt. Die Enzymkonzentration beträgt dabei 10 g/l. Nach kurzem Schütteln entsteht daraus ein zweiphasiges flüssiges System, wobei der Absetzvorgang durch Zentrifugation beschleunigt werden kann.

Werden in die Hexanphase ca. 10 ml Decanol eingespritzt, so ist nach ca. 2 Minuten die Bildung von NADH gemäß der Reaktionsgleichung

Decanol + NAD⁺ ⇔ Decanal + NADH + H⁺

spektralphotometrisch bei einer Wellenlänge von 339 nm in der Tensidphase nachweisbar. Nach etwa 1 Stunde ist auch das gebildete Decanal in der Hexanphase gut gaschromatographisch nachweisbar.

### Beispiel 3:

Es wird in gleicher Weise verfahren wie in Beispiel 2. An Stelle der 5 ml wäßriger Enzymlösung wird dem System eine Suspension von Saccaromyces cerevisiae der Konzentration 10 g/l (bezogen auf Trockenmasse) zugesetzt. Bei Zugabe von 15 ml Propanal in die Hexanphase, ist entsprechend Beispiel 2 die Bildung von NADH in der Tensidphase und von Propanal in der Hexanphase nachweisbar.

### Beispiel 4:

Es wird eine 10 gewichtsprozentige Lösung von Tetraethylenglykoldodecylether in einem organischen Lösungsmittelgemisch, bestehend aus 90 Volumenprozent Hexan und 10 Volumenprozent Aceton, hergestellt. Zu 143 ml dieser Stammlösung werden 6 ml einer 21 mM wäßrigen NAD⁺ -Lösung gegeben. Anschließend werden 3 ml einer wäßrigen Lösung von Hefe-Alkoholdehydrogenase der Konzentration 10 g/l zugespritzt. Nach kurzem Schütteln und anschließender Zentrifugation entsteht ein zweiphasiges flüssiges System. Werden in die Hexanphase 200 ul Benzylalkohol eingespritzt, so ist nach etwa 3 Minuten die Bildung von NADH gemäß der Reaktionsgleichung

Benzlylalkohol + NAD⁺ ⇄ Benzaldehyd + NADH + H⁺

spektralphotometrisch bei einer Wellenlänge von 365 nm in der unteren Phase, der Tensidphase, nachweisbar. Die Zunahme der Benzaldehydkonzentration in der Hexanphase kann ebenfalls spektralphotometrisch bei einer Wellenlänge von 325 nm verfolgt werden.

### Beispiel 5:

Es wird eine 10 gewichtsprozentige Lösung von Heptaoxyethylentetradecylether in Benzin (Kₚ = 42 bis 80^{o} C) hergestellt. In 1 l dieser Lösung werden zuerst 10 ml einer 21 mM wäßrigen NAD⁺-Lösung und danach 10 ml einer wäßrigen Hefe-Alkoholdehydrogenase-Lösung der Konzentration 10 g/l gegeben. Nach kurzem Schütteln und Stehenlassen entsteht ein zweiphasiges flüssiges System. Werden in die Benzinphase 10 ml n-Dodecanol gegeben, ist nach ca. 5 Minuten die Bildung von NADH gemäß der Reaktionsgleichung.

n-Dodecanol + NAD⁺ ⇄ n-Dodecanal + NADH + H⁺

in der Tensidphase spektralphotometrisch bei einer Wellenlänge von 339 nm nachweisbar. Nach ca. 10 Minuten läßt sich auch das entstehende n-Dodecanal in der Hexanphase gaschromatographisch gut nachweisen.

### Beispiel 6:

Es wird eine 10 gewichtsprozentige Lösung von Polyoxyethylen (7) nonylphenylether in Hexan hergestellt. Zu 1 l dieser Lösung werden zunächst 9 ml einer 21 mM wäßrigen NAD⁺ -Lösung und anschließend 4 ml einer wäßrigen Hefe-Alkoholdehydrogenaselösung mit einer Enzymkonzentration von 10 g/l gegeben. Nach kurzem Schütteln und Zentrifugation entsteht ein zweiphasiges flüssiges System. Gibt man 10 ml Zimtalkohol zu diesem System, so läßt sich die Bildung von NADH in der Tensidphase innerhalb von ca. 10 Minuten gut nachweisen. Der gebildete Zimtaldehyd kann ebenfalls nach ca. 10 Minuten in der Hexanphase spektralphotometrisch bei einer Wellenlänge von 325 nm nachgewiesen werden.

### Beispiel 7:

Es wird eine 10 gewichtsprozentige Lösung von Tetraethylenglykoldodecylether in Hexan hergestellt. In einer Lösung von 20 Volumenprozent Methylglykol in Wasser werden einmal 10 g/l NAD⁺ und zum anderen 10 g/l Hefe-Alkoholdehydrogenase gelöst.

Zu 1 ,36 l Tensidlösung werden 100 ml der NAD⁺ - und 50 ml der Hefe-Alkoholdehydrogenaselösung gegeben. Nach leichtem Schütteln entsteht ein zweiphasiges flüssiges System, das sich nach kurzem Stehen gut absetzt. Gibt man in die Hexanphase ca. 10 ml Butanol, läßt sich das entstehende NADH in der Tensidphase nach ca. 5 Minuten gut nachweisen. Das entstehende Butanal läßt sich in der Hexanphase nach ca. 10 Minuten gaschromatographisch nachweisen.

### Beispiel 8:

Es wird eine Lösung von 10 Volumenprozent Aceton in Hexan hergestellt. Damit wird eine 10 gewichtsprozentige Lösung von Tetraethylenglykoldodecylether hergestellt. Zu 1 l dieser Lösung werden zuerst 20 ml einer 21 mM wäßrigen NAD⁺-Lösung und dann 20 ml einer wäßrigen Hefe-Alkoholdehydrogenaselösung mit einer Enzymkonzentration von 10 g/l gegeben. Nach leichtem Schütteln entsteht ein zweiphasiges flüssiges System.

Werden in die Hexan-Aceton-Phase 10 ml Butanol gegeben, ist nach ca. 5 Minuten die Bildung von NADH in der Tensidphase nachweisbar. Das entstehende Butanal läßt sich nach ca. 10 Minuten in der Hexan-Aceton-Phase gaschromatographisch nachweisen.

In gleicher Weise reagieren Ethanol, Hexanol, Heptanol, Decanol. Gibt man nach dieser Reaktion 10 ml Zimtaldehyd zu, wird in der Tensidphase das entstehende NADH wieder zu NAD⁺ zurückgebildet. Die Abnahme des Zimtaldehyds läßt sich in beiden Phasen spektralphotometrisch bei einer Wellenlänge von 325 nm nachweisen.

Das Schema veranschaulicht das Gesamtsystem.

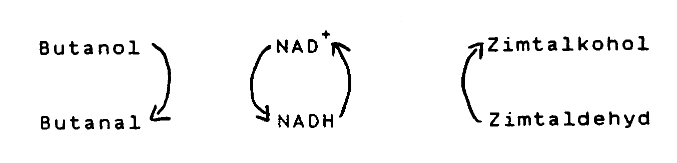

Der entstehende Zimtalkohol kann in einfacher Weise, z.B. mit Wasser, aus der abgetrennten Hexanphase extrahiert werden.

### Beispiel 9:

Es wird eine Tensidlösung entsprechend Beispiel 6 hergestellt. Zu 1 l dieser Lösung werden 20 ml einer Natriumphosphatpufferlösung (5 mM; pH 7,5), die 1,0 Gewichtsprozent Meerrettich-Peroxidase und 3, 10⁻³ mol/l Wasserstoffperoxid enthält, gegeben. Nach Schütteln entsteht ein zweiphasiges flüssiges System. Werden dem System 10 g p-Phenylendiamin zugegeben, so läßt sich das nach der Reaktionsgleichung

p-Phenylendiamin + H₂O₂ → p-Phenylendiimin + 2 H₂O

entstehende Diimin spektralphotometrisch bei einer Wellenlänge von 485 nm nach ca. 5 Minuten in der Tensidphase und nach ca. 10 Minuten auch in der Hexanphase nachweisen.

### Beispiel 10:

In 2 ml einer 10 gewichtsprozentigen Lösung von Tetraethylenglykoldodecylether werden 150 »l Methylglykol eingespritzt. Anschließend werden 100 »l einer wäßrigen Lösung von Dipeptidyl-Peptidase IV (E.C. 3.4. 14.5) in Natriumphosphatpufferlösung (5 mM; pH 7,5) zugegeben. Die Enzymkonzentration beträgt dabei 0,4 g/l. Nach Schütteln und Stehenlassen (3 Minuten) entsteht ein zweiphasiges flüssiges System. Werden zu diesem System ca. 3 mg Glycyl-Prolin-4-Nitroanilid in fester Form als Substrat zugegeben, so ist nach 2 Minuten die Bildung von gelb gefärbtem p-Nitroanilin in der Tensidphase zu erkennen. Nach ca. 7 Minuten erscheint das Reaktionsprodukt auch in der überstehenden Hexanphase. Ein quantitativer Nachweis kann spektralphotometrisch bei einer Wellenlänge von 390 nm erfolgen.

## Patentansprüche

1. Verfahren zur biokatalytischen Umsetzung von organischen Substanzen mit integrierter extraktiver Aufarbeitung,
dadurch gekennzeichnet,
daß eine den Biokatalysator enthaltende, aus Wasser, Tensid und organischem Lösungsmittel gebildete Tensid- oder Mittelphase im Kontakt mit der koexistierenden organischen Lösungsmittelphase, die als Extraktionsmittel für ein entstehendes hydrophobes Produkt dient, eingesetzt wird.

2. Verfahren nach Anspruch 1,
dadurch gekennzeichnet,
daß eine Tensidphase eingesetzt wird, die im ternären Zustandsdiagramm Tensid/Wasser/organisches Lösungsmittel mit der reversmizellaren Phase ein einheitliches Zustandsgebiet ausbildet.

3. Verfahren nach Anspruch 1 oder 2,
dadurch gekennzeichent,
daß als Biokatalysatoren Enzyme, pro- und eukariotische Zellen oder Enzyme in Kombination mit Zellen eingesetzt werden.

4. Verfahren nach einem der Ansprüche 1 bis 3,
dadurch gekennzeichent,
daß Substrate, die nicht in der organischen Lösungsmittelphase löslich sind, in fester Form zugegeben und direkt in der Tensidphase gelöst werden.

5. Verfahren nach einem der Ansprüche 1 bis 4,
dadurch gekennzeichnet,
daß das Substrat als Gas zugeführt und/oder das Produkt als Gas abgeführt wird.

## Claims

1. A process for the biocatalytic reaction of organic substances with integrated working-up by extraction, characterized in that a surfactant or middle phase of water, surfactant and organic solvent, which contains the biocatalyst, in contact with the co-existing organic solvent phase serving as extractant for any hydrophobic product formed is used.

2. A process as claimed in claim 1, characterized in that a surfactant phase which forms a single phase region with the reverse-micellar phase in the surfactant/water/organic solvent ternary phase diagram is used.

3. A process as claimed in claim 1 or 2, characterized in that enzymes, pro- and eukariotic cells or enzymes in combination with cells are used as the biocatalysts.

4. A process as claimed in any of claims 1 to 3, characterized in that substrates insoluble in the organic solvent phase are added in solid form and are directly dissolved in the surfactant phase.

5. A process as claimed in any of claims 1 to 4, characterized in that the substrate is introduced as a gas and/or the product is removed as a gas.

## Revendications

1. Procédé pour la mise en réaction biocatalytique de substances organiques, à traitement extractif intégré, caractérisé en ce que l'on met en oeuvre une phase tensioactive ou intermédiaire contenant le biocatalyseur, formée à partir d'eau, d'un agent tensioactif et d'un solvant organique, en contact avec la phase de solvant organique coexistante, qui fait office d'agent d'extraction pour un produit hydrophobe qui se forme.

2. Procédé selon la revendication 1, caractérisé en ce que l'on met en oeuvre une phase tensioactive qui constitue un domaine d'état homogène dans le diagramme d'équilibre ternaire agent tensioactif/eau/solvant organique avec la phase micellaire réverse.

3. Procédé selon la revendication 1 ou 2, caractérisé en ce que comme biocatalyseurs, on met en oeuvre des enzymes, des cellules pro- et eucaryotes ou encore des enzymes en combinaison avec des cellules.

4. Procédé selon l'une quelconque des revendications 1 à 3, caractérisé en ce que l'on ajoute sous forme solide des substrats qui ne sont pas solubles dans la phase de solvant organique, et on les dissout directement dans la phase tensioactive.

5. Procédé selon l'une quelconque des revendications 1 à 4, caractérisé en ce que le substrat est acheminé sous forme de gaz et/ou le produit est évacué sous forme de gaz.
